# EUROPEAN PATENT APPLICATION

(11) **EP 4 729 009 A1**
(43) Date of publication of application: **22.04.2026**
(21) Application number: 25208925.5
(22) Date of filing: 15.10.2025
(51) Int. Cl.: A61B 18/14

(54) **DETECTING TISSUE TEAR AND PROVIDING OPERATOR FEEDBACK**

(30) Priority: 15.10.2024 US 202418915889
(71) Applicant: Cilag GmbH International, 6300 Zug (CH)
(72) Inventor: Murphy, Joseph Paul, Cincinnati, 45242 (US)
(74) Representative: Carpmaels & Ransford LLP

(57) **Abstract**

A surgical system is disclosed including an electrosurgical instrument and a controller operable to monitor a current provided to the electrosurgical instrument from a power source, detect the current reaching or exceeding a current threshold, compare a rate of power provided to the electrosurgical instrument from the power source to a rate of power threshold based on the current reaching or exceeding the current threshold, initiate a timer to measure elapsed time based on the rate of power being less than the rate of power threshold, and perform an action based on the elapsed time reaching a time threshold.

## Description

### BACKGROUND

The present disclosure relates to surgical systems and, more particularly, bipolar forceps utilized in surgical procedures.

Bipolar forceps are utilized in a variety of electrosurgical procedures. While utilizing the bipolar forceps, there is a chance that the tips of the electrodes may tear through or penetrate patient tissue as bipolar energy is being provided thereto. When this occurs, the electrodes may make electrical contact, causing current to flow through the bipolar forceps, rather than the patient tissue, thereby resulting in no tissue effect.

The lack of tissue effect can lead to frustration and confusion when the bipolar forceps do not seem to be working properly. Accordingly, improved systems and methods for detecting when there is tissue tear when utilizing bipolar forceps are desired.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of a computer-implemented interactive surgical system, in accordance with at least one aspect of the present disclosure.
FIG. 2 is a diagram of various modules, including an energy module, an evacuation module, and an insufflation module, and other components that are combinable to customize modular surgical systems, in accordance with at least one aspect of the present disclosure.
FIG. 3 is the energy module of FIG. 2 and various surgical instruments usable therewith, in accordance with at least one aspect of the present disclosure.
FIG. 4A is a first illustrative modular surgical system configuration including a header module and a display screen that renders a graphical user interface (GUI) for relaying information regarding modules connected to the header module, in accordance with at least one aspect of the present disclosure.
FIG. 4B is an isometric view of the modular surgical system shown in FIG. 4A mounted to a cart, in accordance with at least one aspect of the present disclosure.
FIG. 5 is a second illustrative modular surgical system configuration including a header module, a display screen, two energy modules, and an evacuation module connected together and mounted to a cart, in accordance with at least one aspect of the present disclosure.
FIG. 6 is a block diagram of a modular surgical system, in accordance with at least one aspect of the present disclosure.
FIG. 7 is a block diagram of a modular surgical system, in accordance with at least one aspect of the present disclosure.
FIG. 8 is a method of controlling the modular surgical system of FIG. 7, in accordance with at least one aspect of the present disclosure.

### DETAILED DESCRIPTION

The present disclosure relates to surgical systems and, more particularly, bipolar forceps utilized in surgical procedures.

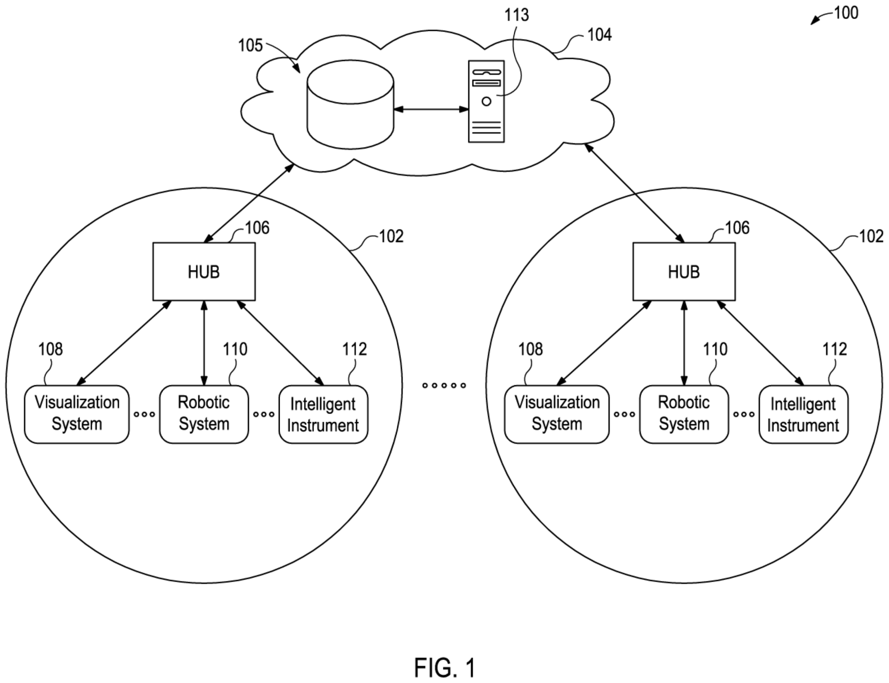

FIG. 1 is a block diagram of a computer-implemented interactive surgical system 100 (hereinafter "the surgical system 100") that may be used in accordance with at least one aspect of the present disclosure. The surgical system 100 includes one or more sub-surgical systems 102 and a cloud-based system (e.g., the cloud 104) that may include a remote server 113 in communication with a storage device 105. Each sub-surgical system 102 includes at least one surgical hub 106 in communication with the cloud 104 that may include a remote server 113.

In one example, as illustrated in FIG. 1, each sub-surgical system 102 includes a visualization system 108, a robotic system 110, and a handheld intelligent surgical instrument 112, which are configured to communicate with one another and/or the hub 106. In some aspects, each sub-surgical system 102 may include an M number of hubs 106, an N number of visualization systems 108, an O number of robotic systems 110, and a P number of handheld intelligent surgical instruments 112, where M, N, O, and P are integers greater than or equal to one. The surgical system 100 is described in more detail in U.S. Patent No. 11,666,368, entitled "METHOD FOR CONSTRUCTING AND USING A MODULAR SURGICAL ENERGY SYSTEM WITH MULTIPLE DEVICES", issued June 6, 2023, and is hereby incorporated by reference in its entirety herein.

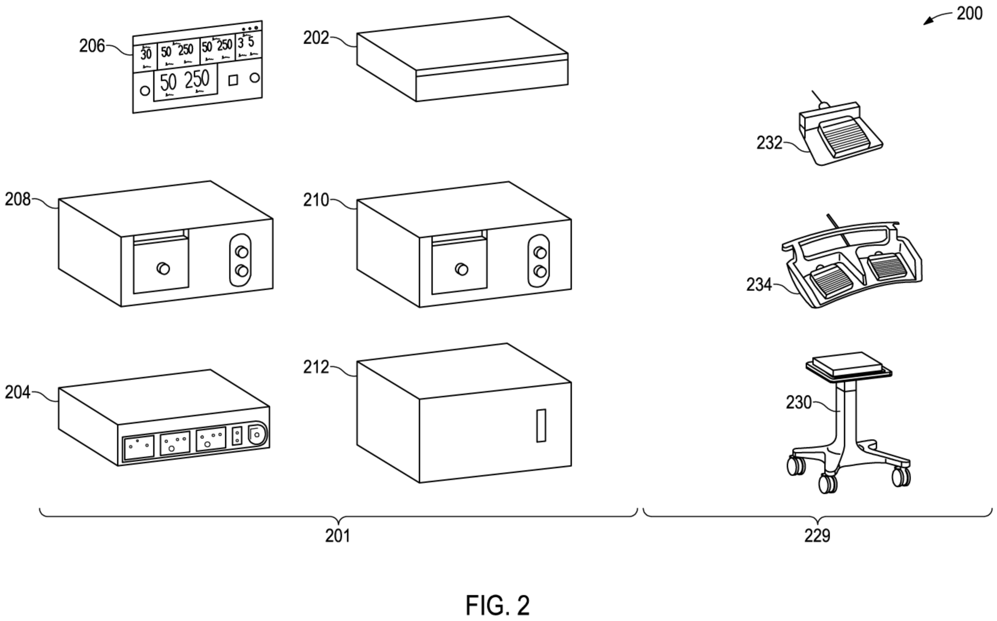

Referring now to FIG. 2, an example surgical hub 106 (FIG. 1) can be embodied as a modular surgical system 200 that can include a variety of different modules 201 that are connectable together in a stacked configuration. In one aspect, the modules 201 can be both physically and communicably coupled together when stacked or otherwise connected together into a singular assembly. Further, the modules 201 can be interchangeably connectable together in different combinations or arrangements. In one aspect, each of the modules 201 can include a consistent or universal array of connectors disposed along their upper and lower surfaces, thereby allowing any module 201 to be connected to another module 201 in any arrangement (except that, in some aspects, a particular module type, such as the header module 202, can be configured to serve as the uppermost module within the stack, for example). In an alternative aspect, the modular surgical system 200 can include a housing that is configured to receive and retain the modules 201. The modular surgical system 200 can also include a variety of different components or accessories that are also connectable to or otherwise associatable with the modules 201.

The modular surgical system 200 can be assembled from a variety of different modules 201, some examples of which are illustrated in FIG. 2. Each of the different types of modules 201 can provide different functionality, thereby allowing the modular surgical system 200 to be assembled into different configurations to customize the functions and capabilities of the modular surgical system 200 (e.g., by customizing the modules 201 that are included in each modular surgical system 200). The modules 201 of the modular surgical system 200 can include, for example, a header module 202 (which can include a display screen 206), an energy module 204, an evacuation module 208, an insufflation module 210, and a visualization module 212.

The modular surgical system 200 can further include a variety of accessories 229 that are connectable to the modules 201 for controlling the functions thereof or that are otherwise configured to work in conjunction with the modular surgical system 200. The accessories 229 can include, for example, a single-pedal footswitch 232, a dual-pedal footswitch 234, and a cart 230 for supporting the modular surgical system 200 thereon. The footswitches 232, 234 can be configured to control the activation or function of particular energy modalities output by the energy module 204, for example.

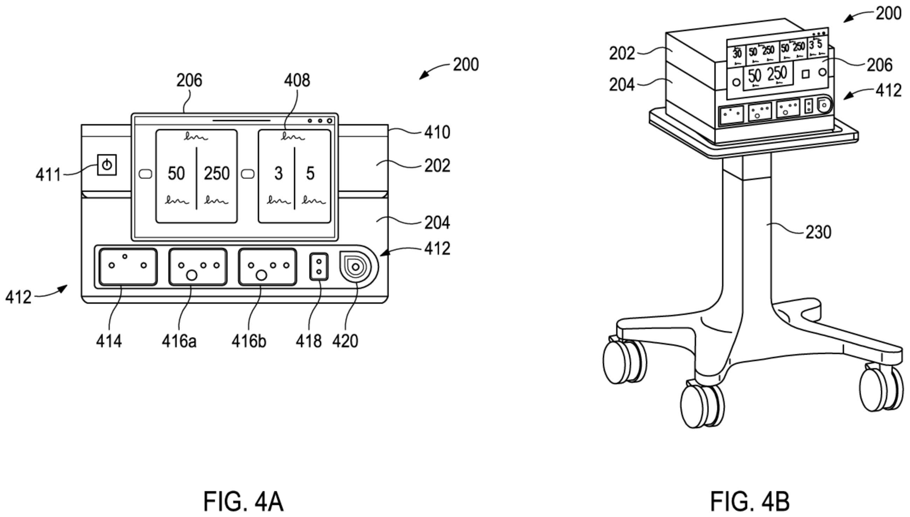

In the depicted aspect, the header module 202 is configured to serve as the top or uppermost module within the modular surgical system stack and can thus lack connectors along its top surface. In another aspect, the header module 202 can be configured to be positioned at the bottom or the lowermost module within the modular surgical system stack (i.e., a "footer" module) and can thus lack connectors along its bottom surface. In yet another aspect, the header module 202 can be configured to be positioned at an intermediate position within the modular surgical system stack and can thus include connectors along both its bottom and top surfaces. The header module 202 can be configured to control the system-wide settings of each module 201 and component connected thereto through physical controls 411 (FIG. 4A) thereon and/or a graphical user interface (GUI) 408 (FIG. 4A) rendered on the display screen 206. Such settings could include the activation of the modular surgical system 200, the volume of alerts, the footswitch settings, the settings icons, the appearance or configuration of the user interface, the surgeon profile logged into the modular surgical system 200, and/or the type of surgical procedure being performed. The header module 202 can also be configured to provide communications, processing, and/or power for the modules 201 that are connected to the header module 202.

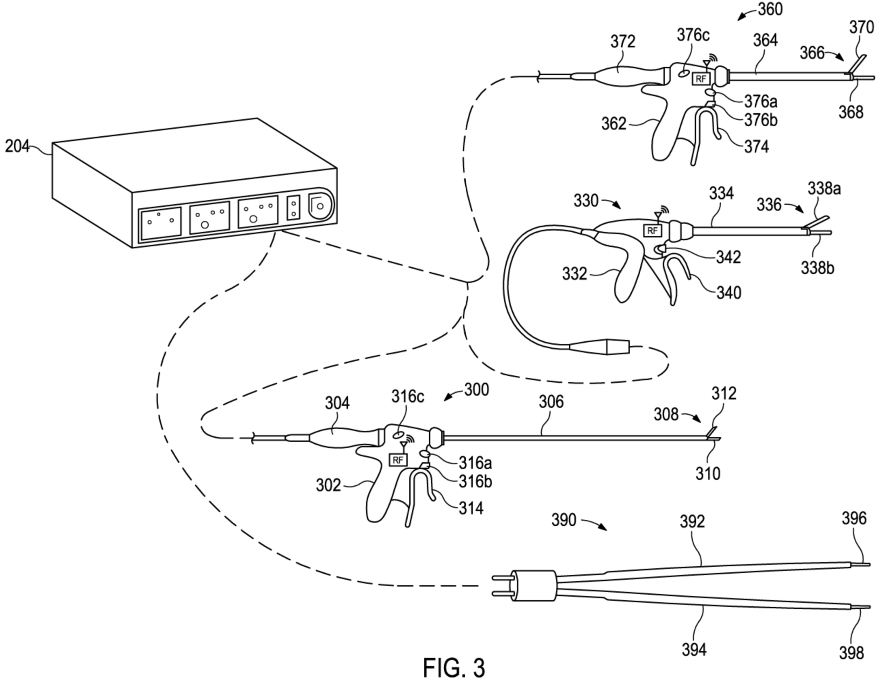

The energy module 204, alternately referred to as a generator module, can be configured to generate one or multiple energy modalities for driving electrosurgical and/or ultrasonic surgical instruments connected thereto. For example, referring to FIG. 3, the generator 204 is configured to drive multiple surgical instruments 300, 330, 360, 390. The first surgical instrument is an ultrasonic surgical instrument 300 and comprises a handpiece 302 (HP), an ultrasonic transducer 304, a shaft 306, and an end effector 308. The end effector 308 comprises an ultrasonic blade 310 acoustically coupled to the ultrasonic transducer 304 and a clamp arm 312. The handpiece 302 comprises a trigger 314 to operate the clamp arm 312 and a combination of toggle buttons 316a, 316b, 316c to energize and drive the ultrasonic blade 310 or other function. The toggle buttons 316a-c can be configured to energize the ultrasonic transducer 304 with the generator 204.

The generator 204 is also configured to drive the second surgical instrument 330, which is an RF electrosurgical instrument and comprises a handpiece 332 (HP), a shaft 334, and an end effector 336. The end effector 336 comprises electrodes in clamp arms 338a, 338b and return through an electrical conductor portion of the shaft 334. The electrodes are coupled to and energized by a bipolar energy source within the generator 204. The handpiece 332 includes a trigger 340 manually actuatable to operate the clamp arms 338a,b, and an energy button 342 to actuate an energy switch to energize the electrodes in the end effector 336.

The generator 204 is also configured to drive the third surgical instrument 360, which is a multifunction surgical instrument 360 and comprises a handpiece 362 (HP), a shaft 364, and an end effector 366. The end effector 366 comprises an ultrasonic blade 368 and a clamp arm 370. The ultrasonic blade 368 is acoustically coupled to an ultrasonic transducer 372. The handpiece 362 includes a trigger 374 to operate the clamp arm 370, and a combination of toggle buttons 376a, 376b, 376c to energize and drive the ultrasonic blade 368 or other function. The toggle buttons 376a-c can be configured to energize the ultrasonic transducer 372 with the generator 204 and energize the ultrasonic blade 368 with a bipolar energy source also contained within the generator 204. Further aspects of the surgical instruments are described in U.S. Patent No. 10,624,691, entitled "TECHNIQUES FOR OPERATING GENERATOR FOR DIGITALLY GENERATING ELECTRICAL SIGNAL WAVEFORMS AND SURGICAL INSTRUMENTS", issued April 21, 2020, which is herein incorporated by reference in its entirety herein.

The generator 204 is also configured to drive the fourth surgical instrument 390, which are bipolar forceps that comprise a first arm 392, a second arm 394, a first electrode 396 at a distal end of the first arm 392, and a second electrode 398 at a distal end of the second arm 394. The first and second arms 392, 394 are graspable by a user to position the first and second electrodes 396, 398 relative to one another. The first and second electrodes 396, 398 are coupled to and energized by a bipolar energy source within the generator 204 and are energized by the same based on a user providing an input to an energy switch, as the single-pedal footswitch 232 or the dual-pedal footswitch 234.

The evacuation module 208 (FIG. 2) can be configured to evacuate smoke, fluid, and/or particulates generated by the application of therapeutic energy to the tissue by one or more of the surgical instruments 300, 330, 360. Example evacuation modules are described in more detail elsewhere herein, as well as in U.S. Patent No. 11,602,393, entitled "SURGICAL EVACUATION SENSING AND GENERATOR CONTROL", issued March 14, 2023, which is hereby incorporated by reference in its entirety herein. The insufflation module 210 (FIG. 2) can be configured to blow air or gas into a body cavity of a patient to inflate it for diagnostic or surgical procedures, thereby providing better visibility and access during procedures.

The visualization module 212 (FIG. 2) can be configured to interface with visualization devices (i.e., scopes) and accordingly provide increased visualization capabilities. Example visualization modules and systems are described in more detail in U.S. Patent No. 11,284,963, entitled "METHOD OF USING IMAGING DEVICES IN SURGERY", issued March 29, 2022, which is hereby incorporated by reference in its entirety herein.

By utilizing modular components, the depicted modular surgical system 200 provides a surgical platform that grows with the availability of technology and is customizable to the needs of the facility and/or surgeons. Further, the modular surgical system 200 supports combo devices (e.g., dual electrosurgical and ultrasonic energy generators) and supports software-driven algorithms for customized tissue effects. Still further, the surgical system architecture reduces the capital footprint by combining multiple technologies critical for surgery into a single system.

The various modular components utilizable in connection with the modular surgical system 200 can include monopolar energy generators, bipolar energy generators, dual electrosurgical/ultrasonic energy generators, display screens, and various other modules and/or other components described elsewhere herein.

Referring now to FIG. 4A, the header module 202 can, in some aspects, include the display screen 206 that renders a GUI 408 for relaying information regarding the modules 201 (FIG. 2) connected to the header module 202. In some aspects, the GUI 408 of the display screen 206 can provide a consolidated point of control of all of the modules 201 making up the particular configuration of the modular surgical system 200. In alternative aspects, the header module 202 can lack the display screen 206, or the display screen 206 can be detachably connected (removably attachable) to a housing 410 of the header module 202. In such aspects, the header module 202 can be communicably couplable to an external system that is configured to display the information generated by the modules 201 of the modular surgical system 200. For example, in robotic surgical applications, the modular surgical system 200 can be communicably couplable to a robotic cart or robotic control console, which is configured to display the information generated by the modular surgical system 200 to the operator of the robotic surgical system. As another example, the modular surgical system 200 can be communicably couplable to a mobile display that can be carried or secured to a surgical staff member for viewing thereby. In aspects utilizing a user interface that is separate from or otherwise distinct from the modular surgical system 200, the user interface can be wirelessly connectable with the modular surgical system 200 as a whole or one or more modules 201 thereof such that the user interface can display information from the connected modules 200 thereon.

Referring still to FIG. 4A, the energy module 204 can include a port assembly 412 including (providing) a number of different ports configured to deliver different energy modalities to corresponding surgical instruments (e.g., surgical instruments 300, 330, 360 of FIG. 3, for example) that are connectable thereto. In the particular aspect illustrated in FIGS. 4A, the port assembly 412 includes a bipolar port 414, a first monopolar port 416a, a second monopolar port 416b, a neutral electrode port 418 (to which a monopolar return pad is connectable), and a combination energy port 420. However, this particular combination of ports is simply provided for illustrative purposes and alternative combinations of ports and/or energy modalities may be possible for the port assembly 412.

As noted above, the modular surgical system 200 can be assembled into different configurations. Further, the different configurations of the modular surgical system 200 can also be utilizable for different surgical procedure types and/or different tasks. For example, FIGS. 4A and 4B illustrate a first illustrative configuration of the modular surgical system 200 including the header module 202 (including the display screen 206) and the energy module 204 connected together. Such a configuration can be suitable for laparoscopic and open surgical procedures, for example. As shown in FIG. 4B, the modular surgical system 200 can be positioned on a cart 230 enabling the modular surgical system 200 to be easily moved (wheeled) around the operating room, for example.

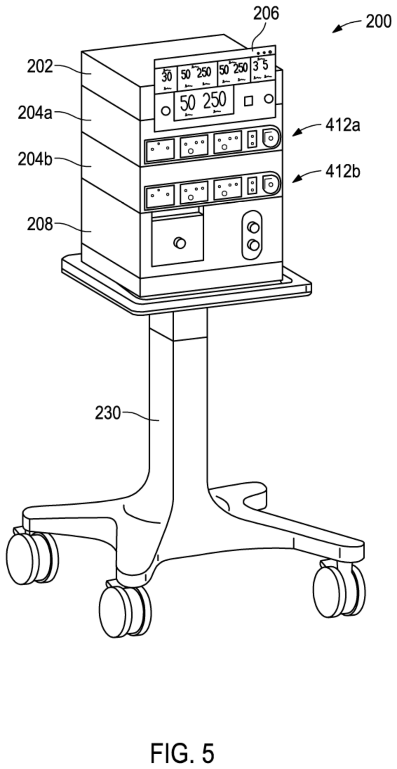

FIG. 5 illustrates a second illustrative configuration of the modular surgical system 200 including the header module 202 (including the display screen 206), a first energy module 204a, a second energy module 204b, and the evacuation module 208 connected together and positioned on the cart 230. In such a configuration, the evacuation module 208 can evacuate smoke, fluid, and/or particulates generated by surgical instruments powered by the energy modules 204a,b.

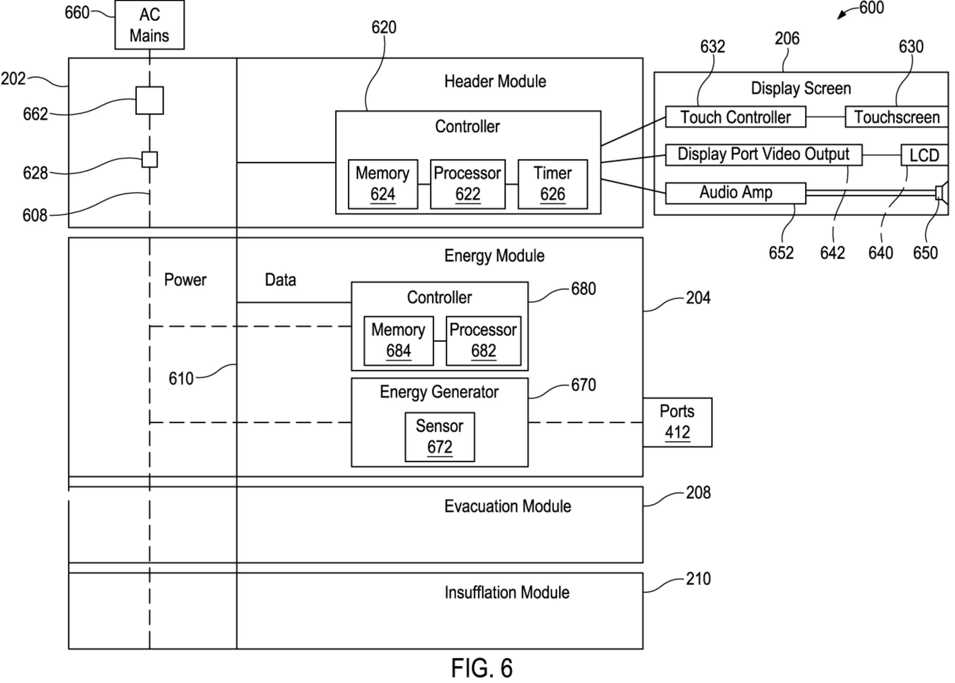

FIG. 6 is a block diagram of an example modular surgical system 600, in accordance with at least one aspect of the present disclosure. As illustrated, the modular surgical system 600 includes the header module 202 (including the display screen 206), the energy module 204 stacked under and coupled to the header module 202, the evacuation module 208 stacked under and coupled to the energy module 204, and the insufflation module 210 stacked under and coupled to the evacuation module 208.

The header module 202 is configured to monitor, control, energize, and provide feedback concerning operation of the modules within the modular surgical system 600, such as the energy module 204, the evacuation module 208, and the insufflation module 210. As illustrated, the header module 202 includes a controller 620 that comprises a processor 622 and a memory 624 storing computer readable instructions executable by the processor 622 to carry out functions and operations of the header module 602. Examples of the memory 624 include, but are not limited to, random access memory (RAM), read-only memory (ROM), computer chips, optical discs (e.g., compact discs (CDs), digital video discs (DVDs), etc.), magnetic disks (e.g., hard disk drives (HDDs), floppy disks, ZIP^{®} disks, etc.), magnetic tape, and solid state storage devices (e.g., memory cards, "flash" media, etc.). As used herein, the term "computer readable medium" refers to any device or system for storing and providing information (e.g., data and instructions) to the processor 622. Examples of computer readable media include, but are not limited to, optical discs, magnetic disks, magnetic tape, solid-state media, and servers for streaming media over networks.

Based on instructions stored in the memory 624, the processor 622 may be configured to control power and data transmissions between the header module 202, the energy module 204, the evacuation module 208, and the insufflation module 210 through a power interface 608 and a data interface 610. For example, the header module 202 can transmit various commands to the energy module 204, the evacuation module 208 (through the energy module 204), and the insufflation module 210 (through the energy module 204 and the evacuation module 208) via the data interface 610. Such commands can be based on user inputs received at the display screen 206 or inputs received by the controller 620 from various sensors communicably coupled to the modular surgical system 600, as discussed elsewhere herein.

As a further example, power may be transmitted to the energy module 204, the evacuation module 208 (through the energy module 204), and the insufflation module 210 (through the energy module 204 and the evacuation module 208) from the header module 202 via the power interface 608. The header module 202 may receive power from an external power source 660 (referred to herein as "AC Mains"), such as a wall outlet, for example. The header module 202 may include an AC/DC converter 662 which receives the AC power from the AC Mains 660 and converts the AC power to DC power. The controller 202 may then distribute the DC power to the energy module 204, the evacuation module 208, and the insufflation module 210 via the power interface 608. The controller 620 may further include a timer 626 for measuring elapsed time. The header module 202 may include a sensor 628, such as a current sensor and/or a power sensor, for example, in operable communication with the controller 620 for measuring current and power along the power interface 608.

As shown in FIG. 6, the energy module 204 may include a controller 680 that comprises a processor 682 and a memory 684 storing computer readable instructions executable by the processor 682 to carry out functions and operations of the energy module 204. The processor 682 and a memory 684 may be similar to processor 622 and memory 624, respectively. The controller 680 may receive power from the AC/DC converter 662 along the power interface 608 and may be in operable communication with controller 620 via the data interface 610.

The energy module 204 may further include an energy generator 670. The energy generator 670 may receive power from the AC/DC converter 662 along the power interface 608 and may be in operable communication with controller 680, such as via a wired or wireless connection. The energy generator 670 may be operable to provide therapeutic energy to one or more surgical instruments, such as the surgical instruments 300, 330, 360, 390, via the port assembly 412, such as via the bipolar port 414 (FIG. 4), the first or second monopolar ports 416a, 416b (FIG. 4), or the combination energy port 420 (FIG. 4), for example. For instance, the energy generator 670 may be energized with DC power provided thereto from the AC/DC converter 662 along the power interface 608. The controller 680 may then receive an input, such as from the controller 620. Based on the input, the controller 680 may control the energy generator 670 to provide therapeutic energy to one or more surgical instruments coupled to the energy module 204 at the port assembly 412. The energy generator 670 may include a sensor 672, such as a current sensor and/or a power sensor, for example, in operable communication with the controller 680 for measuring current and/or power provided by the energy generator 670. The sensor 672 may also comprise an impedance sensor for measuring the impedance of tissue grasped by one of the surgical instruments.

As shown in FIG. 6, the display screen 206 includes a touchscreen 630 coupled to a touch controller 632. The touch controller 632 is coupled to the controller 620 to read inputs, such as user inputs, from the touchscreen 630. The controller 620 drives an LCD display 640 through a display/port video output signal 642. The controller 620 is further coupled to an audio amplifier 652 to drive one or more speakers 650.

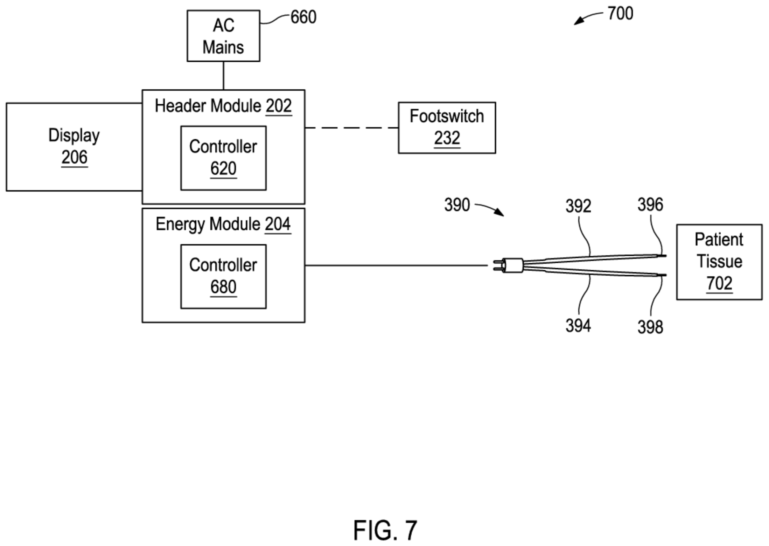

FIG. 7 is a schematic block diagram of another example modular surgical system 700, in accordance with at least one aspect of the present disclosure. The modular surgical system 700 may be similar in some respects to the modular surgical system 600 of FIG. 6, and therefore may be best understood with reference thereto. As illustrated, for example, the modular surgical system 700 includes the header module 202 that includes the controller 620, the display screen 206 coupled to (in communication with) the header module 202, and the energy module 204 stacked under and communicably coupled to the header module 202 and including the controller 680. The modular surgical system 700 may include additional modules described elsewhere herein, such as the insufflation module 210 and/or the evacuation module 208, for example. The modular surgical system 700 may further include the bipolar forceps 390 described herein with reference to FIG. 3, where the bipolar forceps 390 are electrically coupled to the energy module 204, such as at the bipolar port 414 (FIG. 4).

In operation, with reference now to FIGS. 6 and 7, a user, such as a surgeon, may desire to provide electrosurgical energy to patient tissue 702 with the electrodes 396, 398 of the bipolar forceps 390. The user may first provide an input to the controller 620 indicative of a mode of operation of the bipolar forceps 390. For instance, the bipolar forceps 390 may be utilized in a first "manual" mode or a second "automatic" mode.

In the manual mode, a user may grasp (or otherwise place a lateral load on) the arms 392, 394 of the bipolar forceps 390 and utilize the same to grasp patient tissue 702 between the ends of the arms 392, 394. When the user desires to provide electrosurgical energy to the patient tissue 702 with the electrodes 396, 398 of the bipolar forceps 390, the user may provide an input to the controller 620, such as via a footswitch 232. Based on the input, the controller 620 may provide an input to the controller 680 of the energy module 204, which may in turn control the energy generator 670 (FIG. 6) to energize the electrodes 396, 398 of the bipolar forceps 390.

In the automatic mode, a user may grasp (or otherwise place a lateral load on) the arms 392, 394 of the bipolar forceps 390. When the user desires to provide electrosurgical energy to the patient tissue 702 with the electrodes 396, 398 of the bipolar forceps 390, the user may grasp the patient tissue 702 between the ends of the arms 392, 394. The controller 680 may automatically detect the patient tissue 702 being grasped by the arms 392, 394 of the bipolar forceps 390. As one example, the sensor 672 may be comprise an impedance sensor and the sensor 672 may detect a change in impedance based on the arms 392, 394 of the bipolar forceps 390 grasping the patient tissue 702. As another example, the arms 392, 394 of the bipolar forceps 390 may include pressure sensors that detect pressure being applied thereto, such as the patient tissue 702 being grasped thereby. Based on the detection of the patient tissue 702 being grasped by the bipolar forceps 390, the controller 680 may automatically control the energy generator 670 to energize the electrodes 396, 398 of the bipolar forceps 390.

While utilizing the bipolar forceps 390, there is a chance that the tips of the electrodes 396, 398 may tear through or penetrate the patient tissue 702 as bipolar energy is being provided thereto. When this occurs, the electrodes 396, 398 may make electrical contact, causing current to flow through the bipolar forceps 390, rather than the patient tissue 702, thereby resulting in no tissue effect.

The lack of tissue effect can lead to frustration and confusion when the bipolar forceps 390 do not seem to be working properly. Accordingly, improved systems and methods for detecting when there is tissue tear when utilizing bipolar forceps 390 are desired.

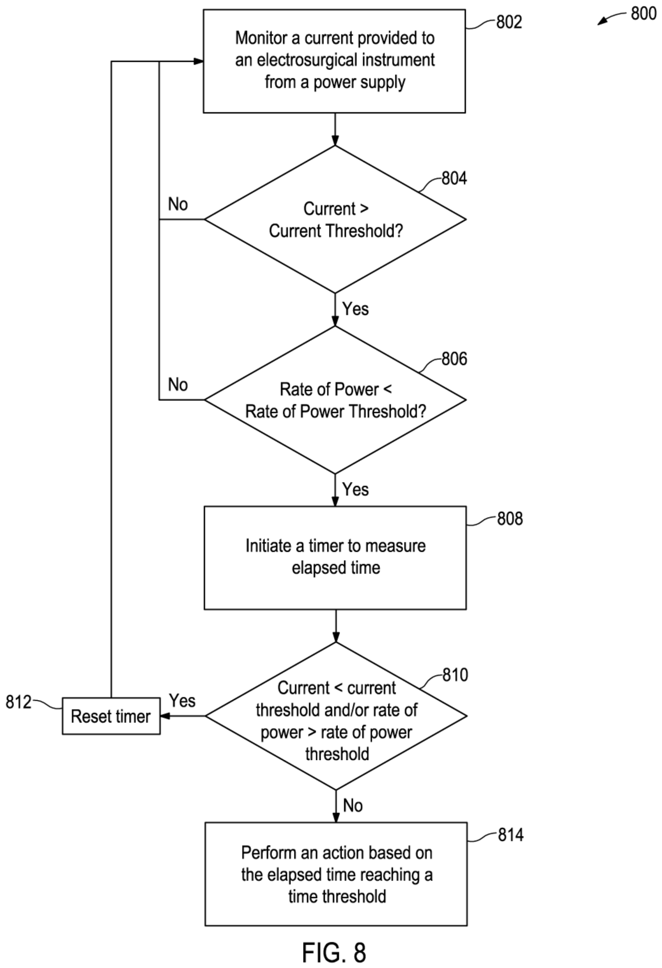

FIG. 8 is a schematic flow chart of an example method 800 of controlling the modular surgical system 700 of FIG. 7, according to at least one aspect of the present disclosure. The method 800 may be embodied as an algorithm stored in the memory 624 (FIG. 6) of the controller 620 (FIG. 7), and may be executable by the processor 622 (FIG. 6) of the controller 620 based on a user providing an input to the controller 620, such as at the touchscreen 630 (FIG. 6) of the controller 620. Alternatively, the algorithm may be stored in the memory 684 (FIG. 6) of the controller 680 (FIG. 7), and may be executable by the processor 682 (FIG. 6) of the controller 680 based on a user providing an input to the controller 680, such as via the touchscreen 630 (FIG. 6).

With reference to FIGS. 6-8, the method 1400 may include monitoring a current provided to an electrosurgical instrument from a power source, as at step 802. For instance, when the user desires to energize the electrodes 396, 398 of the bipolar forceps 390, a user may provide an input to the controller 620, such as via a footswitch 232. Based on the input, the controller 620 may provide an input to the controller 680 of the energy module 204, which may control the energy generator 670 to energize the electrodes 396, 398 of the bipolar forceps 390. As the energy generator 670 provides energy to the bipolar forceps 390, the controller 640 may simultaneously monitor the current provided to the bipolar forceps 390, such as with the sensor 672.

The method 800 may further include comparing the measured current to a current threshold, as at step 804. For instance, the memory 644 of the controller 640 may include (have stored therein) a current threshold, and the controller 640 may be operable to compare the monitored (measured) current to the current threshold, such as continuously, periodically, or at predefined intervals. Based on the current being below the current threshold, the controller 640 may loop back to step 802.

The method 800 may further include comparing a rate of power provided to the electrosurgical instrument to a rate of power threshold, as at step 806. For instance, based on the controller 640 detecting that the current has reached or exceeded the current threshold, the controller 640 may assume (determine) that the patient tissue 702 being operated on by the bipolar forceps 390 has torn (or is penetrated), thereby resulting in a short circuit between the electrodes 396, 398 that has caused the current to reach or exceed the current threshold. To validate the assumption (determination), the controller 640 may proceed to compare a rate of power provided by the energy generator 670 to the bipolar forceps 390 to a rate of power threshold, which may also be stored in the memory 664. The controller 640 may monitor the absolute value of the rate of power provided to the bipolar forceps 390, such as with the sensor 672. Based on the absolute value of the rate of power being above the rate of power threshold, the controller 680 may conclude that the patient tissue 702 has not torn. Accordingly, the controller 680 may loop back to step 802.

The method 800 may further include initiating a timer to measure elapsed time, as at step 808. For instance, based on the controller 680 detecting that the absolute value of the rate of power is below the rate of power threshold, the controller 680 may have additional confidence in the assumption that the patient tissue 702 has torn (or has been penetrated). To further validate the assumption, the controller 680 may provide an input to the controller 620, which may in turn proceed to initiate the timer 626 to measure an elapsed amount of time that the current is at or above the current threshold and the absolute value of the rate of power is below the rate of power threshold.

The method 800 may further include comparing the current to the current threshold and/or the rate of power to the rate of power threshold, as at step 810. For instance, as the timer 626 is measuring elapsed time, the controller 680 may monitor the current and/or rate of power against their respective thresholds, such as continuously, periodically, or at predefined intervals. Based on the current dropping below the current threshold and/or the rate of power increasing above the rate of power threshold, the controller 680 may conclude that the patient tissue 702 did not tear. Accordingly, the controller 620 may reset the timer 626, as at step 812, and the controller 680 may loop back to step 802.

The method 800 may further include performing an action based on the elapsed time reaching a time threshold, as at step 814. For instance, the controller 680 may detect that the current was maintained above the current threshold and the absolute value of the rate of power was maintained below the rate of power threshold for a threshold amount of time (time threshold), which may be stored in the memory 644 or the memory 624. Based on the detection, the controller 680 may conclude that the patient tissue 702 has torn and, therefore, may proceed with performing an action. This action may include providing an input to the controller 620, which, in response, may generate (provide) an alert on the display 206, such as a message to the user indicating that the patient tissue 702 has torn and the bipolar forceps 390 should be repositioned, generate (provide) an audible alert via the speaker 650, generate a visible alert (e.g., a light), store, in one of both of the memories 624, 684, a record of the tissue tear, which may be used as a metric for the user (surgeon) for future improvement, or cease to provide therapeutic energy to the bipolar forceps 390 from energy generator 670, or combinations thereof.

Accordingly, the foregoing system and method may detect when there is tissue tear when utilizing an electrosurgical instrument, such as the bipolar forceps 390, thereby alleviating user frustration when it appears that the electrosurgical instrument does not seem to be working properly.

Embodiments disclosed herein include:
A. A surgical system comprising an electrosurgical instrument and a controller operable to monitor a current provided to the electrosurgical instrument from a power source, detect the current reaching or exceeding a current threshold, compare a rate of power provided to the electrosurgical instrument from the power source to a rate of power threshold based on the current reaching or exceeding the current threshold, initiate a timer to measure elapsed time based on the rate of power being less than the rate of power threshold, and perform an action based on the elapsed time reaching a time threshold.
B. A surgical system comprising an energy module operably coupled to an electrosurgical instrument and a controller, operable to compare a current provided to the electrosurgical instrument from the energy module to a current threshold, compare a rate of power provided to the electrosurgical instrument from the energy module to a rate of power threshold, initiate a timer to measure elapsed time based on the current being at or above the current threshold and the absolute value of the rate of power being below the rate of power threshold, and perform an action based on the elapsed time reaching a time threshold.
C. A non-transitory computer-readable medium storing instructions that, when executed by a processor, cause the processor to compare a current provided to an electrosurgical instrument from a power source to a current threshold, compare a rate of power provided to the electrosurgical instrument from the power source to a rate of power threshold, initiate a timer to measure elapsed time based on the current being at or above the current threshold and the absolute value of the rate of power being below the rate of power threshold, and perform an action based on the elapsed time reaching a time threshold.

Each of embodiments A and B may have one or more of the following additional elements in any combination: Element 1: wherein the electrosurgical instrument comprises bipolar forceps. Element 2: further comprising a display, wherein the action comprises providing an alert on the display. Element 3: further comprising a speaker, wherein the action comprises providing an audible alert via the speaker. Element 4: wherein the action comprises ceasing to provide energy to the electrosurgical instrument. Element 5: wherein the controller is further operable to detect the current dropping below the current threshold as the timer measures elapsed time and reset the timer based on the current dropping below the current threshold. Element 6: wherein the controller is further operable to detect the rate of power provided to the electrosurgical instrument increasing above the rate of power threshold as the timer measures elapsed time and reset the timer based on the rate of power increasing above the rate of power threshold.

By way of non-limiting example, exemplary combinations applicable to A, B, and C include: Element 1 with Element 2; Element 1 with Element 3; Element 1 with Element 4; Element 1 with Element 5; Element 1 with Element 6; Element 1 with two or more of Elements 2-6; Element 2 with Element 3; Element 2 with Element 4; Element 2 with Element 5; Element 2 with Element 6; Element 2 with two or more of Elements 1 and 3-6; Element 3 with Element 4; Element 3 with Element 5; Element 3 with Element 6; Element 3 with two or more of Elements 1, 2, and 4-6; Element 4 with Element 5; Element 4 with Element 6; Element 4 with two or more of Elements 1-3, 5, and 6; Element 5 with Element 6; Element 5 with Elements 1-4 and 6; Element 6 with two or more of Elements 1-5.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The use of directional terms such as above, below, upper, lower, upward, downward, left, right, and the like are used in relation to the illustrative embodiments as they are depicted in the figures, the upward direction being toward the top of the corresponding figure and the downward direction being toward the bottom of the corresponding figure.

## Claims

1. A surgical system, comprising:
an electrosurgical instrument; and
a controller operable to:
monitor a current provided to the electrosurgical instrument from a power source;
detect the current reaching or exceeding a current threshold;
compare a rate of power provided to the electrosurgical instrument from the power source to a rate of power threshold based on the current reaching or exceeding the current threshold;
initiate a timer to measure elapsed time based on the rate of power being less than the rate of power threshold; and
perform an action based on the elapsed time reaching a time threshold.

2. The surgical system of Claim 1, wherein the action comprises ceasing to provide energy to the electrosurgical instrument.

3. A surgical system, comprising:
an energy module operably coupled to an electrosurgical instrument; and
a controller, operable to:
compare a current provided to the electrosurgical instrument from the energy module to a current threshold;
compare a rate of power provided to the electrosurgical instrument from the energy module to a rate of power threshold;
initiate a timer to measure elapsed time based on the current being at or above the current threshold and the absolute value of the rate of power being below the rate of power threshold; and
perform an action based on the elapsed time reaching a time threshold.

4. The surgical system of Claim 3, wherein the action comprises ceasing to provide energy to the electrosurgical instrument from the energy module.

5. The surgical system of any preceding Claim, further comprising a display, wherein the action comprises providing an alert on the display.

6. The surgical system of any preceding Claim, further comprising a speaker, wherein the action comprises providing an audible alert via the speaker.

7. The surgical system of any preceding Claim, wherein the controller is further operable to:
detect the current dropping below the current threshold as the timer measures elapsed time; and
reset the timer based on the current dropping below the current threshold.

8. The surgical system of any preceding Claim, wherein the controller is further operable to:
detect the rate of power provided to the electrosurgical instrument increasing above the rate of power threshold as the timer measures elapsed time; and
reset the timer based on the rate of power increasing above the rate of power threshold.

9. The surgical system of any preceding Claim, wherein the electrosurgical instrument comprises bipolar forceps.

10. A non-transitory computer-readable medium storing instructions that, when executed by a processor, cause the processor to:
compare a current provided to an electrosurgical instrument from a power source to a current threshold;
compare a rate of power provided to the electrosurgical instrument from the power source to a rate of power threshold;
initiate a timer to measure elapsed time based on the current being at or above the current threshold and the absolute value of the rate of power being below the rate of power threshold; and
perform an action based on the elapsed time reaching a time threshold.

11. The non-transitory computer-readable medium of Claim 10, wherein the action comprises providing an alert on a display.

12. The non-transitory computer-readable medium of Claim 10 or Claim 11, wherein the action comprises providing an audible alert via a speaker.

13. The non-transitory computer-readable medium of any one of Claims 10 to 12, wherein the action comprises ceasing to provide energy to the electrosurgical instrument from the power source.

14. The non-transitory computer-readable medium of any one of Claims 10 to 13, further storing instructions that, when executed by the processor, cause the processor to:
detect the current dropping below the current threshold as the timer measures elapsed time; and
reset the timer based on the current dropping below the current threshold.

15. The non-transitory computer-readable medium of any one of Claims 10 to 14, further storing instructions that, when executed by the processor, cause the processor to:
detect the rate of power provided to the electrosurgical instrument increasing above the power threshold as the timer measures elapsed time; and
reset the timer based on the rate of power increasing above the power threshold.
